# EUROPÄISCHE PATENTSCHRIFT

(11) **EP 0 930 293 B1**
(45) Veröffentlichungstag und Bekanntmachung des Hinweises auf die Patenterteilung: **29.09.2004**
(21) Anmeldenummer: 99100146.2
(22) Anmeldetag: 08.01.1999
(51) Int. Cl.: C07C 231/12, C07C 233/05, B01J 29/40, B01J 29/00

(54) **Verfahren zur Herstellung von N-Alkenylcarbonsäureamiden**
Process for the preparation of N-alkenyl carboxylic acid amide
Procédé pour la préparation de N-alkenyl-carboxyamides

(30) Priorität: 16.01.1998 DE 19801549
(43) Veröffentlichungstag der Anmeldung: 21.07.1999
(73) Patentinhaber: BASF AKTIENGESELLSCHAFT, 67056 Ludwigshafen (DE)
(72) Erfinder: Ebel, Klaus, Dr., 68623 Lampertheim (DE); Eiermann, Matthias, Dr., 67117 Limburgerhof (DE); Narbeshuber, Thomas, Dr., 49477 Ibbenbüren (DE); Gehrer, Eugen, Dr., 67069 Ludwigshafen (DE)
(74) Vertreter: Isenbruck, Günter, Dr.

(56) Entgegenhaltungen:
- EP-A- 0 232 712
- EP-A- 0 701 998
- US-A- 5 569 770

## Beschreibung

Die Erfindung betrifft ein Verfahren zur Herstellung von N-Alkenylcarbon-säureamiden durch Dehydratisierung von N-(2-Hydroxyalkyl)carbonsäureamiden und/oder Diethern davon in Gegenwart eines Katalysators.

N-Alkenylcarbonsäureamide werden vielfach in technischen Produkten eingesetzt. Beispielsweise wird N-Vinylpyrrolidon (NVP) zur Herstellung von Poly(N-vinylpyrrolidon) (PVP) eingesetzt. PVP wird beispielsweise als Haarsprayzusatz, als PVP-Iod-Komplex in Heilsalben, als Komponente schmelzextrudierter Tabletten oder beim Bierbrauen als Klärmittel eingesetzt. NVP kann nach dem Reppe-Verfahren durch Vinylierung von Pyrrolidon mit Acetylen hergestellt werden. Der technische Umgang mit Acetylen, das auch zur Herstellung des eingesetzten Pyrrolidons verwendet wird, ist jedoch wegen dessen Reaktivität aufwendig und risikobehaftet.

Ein alternativer Zugang zu NVP besteht in der Dehydratisierung von N-(2-Hydroxyethyl)pyrrolidon (HEP), das aus den leicht zugänglichen Komponenten Butyrolacton und Monoethanolamin herstellbar ist. Eine Vielzahl derartiger Verfahren ist beschrieben.

In der DE-A-2 135 211 ist ein Verfahren zur Herstellung von N-Vinylpyrrolidon beschrieben, wobei in Gegenwart eines Oxids von Zirkonium, Thorium, Cer, Zink, Chrom oder Zink/Chrom als Katalysator gearbeitet wird.

Gemäß US 2,669,570 wird aktiviertes Aluminiumoxid als Dehydratisierungskatalysator eingesetzt.

Gemäß EP-A-0 608 690 kommt mit Phosphorsäure behandeltes Siliciumdioxid oder mit Lantannitrat und Phosphat behandelte Diatomeenerde zum Einsatz.

Gemäß EP-A-0 701 998 wird mit einem Alkalisalz dotiertes Siliciumdioxid eingesetzt.

Die vorstehend angegebenen Verfahren müssen teilweise unter vermindertem Druck durchgeführt werden oder weisen noch zu geringe Selektivitäten auf.

Aufgabe der vorliegenden Erfindung ist die Bereitstellung eines Verfahrens zur Herstellung von N-Alkenylcarbonsäureamiden durch Dehydratisierung von N-(2-Hydroxyalkyl)carbonsäureamiden und/oder Diethern davon in Gegenwart eines Katalysators, wobei unter Normaldruck hohe Umsätze und Selektivitäten, insbesondere bei der Synthese von NVP aus HEP möglich sind.

Die Aufgabe wird erfindungsgemäß gelöst durch Einsatz von Molekularsieben, insbesondere Zeolithen, als Katalysator in der vorstehenden Umsetzung, wobei das Verfahren kontinuierlich durchgeführt wird und die nicht dehydratisierten N-(2-Hydroxyalkyl)carbonsäure amide und die als Nebenprodukt gebildeten Diether in die Dehydratisierung zurückgeführt werden.

Katalysatoren auf Molekularsiebbasis sind besonders bevorzugt für die hochselektive Darstellung von NVP aus HEP geeignet, wenn das Modul, das heißt das Verhältnis von Silicium, berechnet als Siliciumdioxid, zu Aluminium, berechnet als Aluminiumoxid, mindestens 140, besonders bevorzugt mindestens 150, insbesondere mindestens 200, speziell mindestens 250 beträgt.

Bei niedrigem Modul, das heißt einer hohen Konzentration an sauren Zentren, findet man eine niedrige NVP-Selektivität zugunsten der dominierenden Bildung des formal aus 2 HEP-Molekülen durch Wasserabspaltung entstehenden Ethers. Dieser kann jedoch selbst als Einsatzstoff zur Herstellung von NVP dienen. Er kann daher zurückgeführt werden und trägt in einer kontinuierlichen Synthese nicht zur Ausbeuteverminderung bei. Als ein in einem kontinuierlichen Verfahren nicht rückführbares und daher weitgehend unerwünschtes Nebenprodukt kann Pyrrolidon entstehen.

Bei hohem Modul, das heißt niedriger Konzentration an sauren Zentren, wird eine hohe Selektivität und Ausbeute in bezug auf NVP bei gleichzeitig niedriger Bildungstendenz für Pyrrolidon beobachtet. Bei Dotierung der Molekularsiebe, insbesondere Zeolithe, mit mindestens einer Alkalimetallverbindung oder Erdalkalimetallverbindung wird die Etherbildung weiter vermindert. Vorzugsweise wird eine Alkalimetalldotierung durchgeführt. Die Dotierung erfolgt vorzugsweise mit Natrium- und/oder Kaliumsalzen. In diesem Fall wird kaum Dieether von HEP gefunden. Zudem kann die Pyrrolidonbildung stark reduziert werden, und die Umsätze sind hoch.

Vorzugsweise werden als Katalysatoren Zeolithe vom Typ ZSM5 (MFI), Y (FAU), LDK10 oder Silikalit eingesetzt. Beispiele sind die Zeolithkatalysatoren MFI, MOR und FAU.

Die erfindungsgemäß verwendeten Katalysatoren werden vorzugsweise als Formkörper in einem stationären oder bewegten Katalysatorbett eingesetzt. Die Formkörper können beispielsweise in Form von Strängen oder Kugeln vorliegen. Zur Herstellung der Formkörper können Hilfsmittel, wie Pural® von Condea, Walocel® von Wolf/Walsrode, Kartoffelstärke oder Aerosil® von Degussa, bevorzugt Walocel und Aerosil eingesetzt werden. Der Anteil der Hilfsmittel am Formkörper kann 0 bis 60, vorzugsweise 10 bis 40 Gew.-% betragen.

Die Dehydratisierung wird vorzugsweise in der Gasphase, gegebenenfalls in Gegenwart eines Trägergases, durchgeführt. Dabei wird vorzugsweise ein stationäres oder ein bewegtes Katalysatorbett, besonders bevorzugt ein stationäres Katalysatorbett eingesetzt.

Die Umsetzung wird vorzugsweise bei Temperaturen von 200 bis 450°C durchgeführt. Bei zu hohen Temperaturen finden sich unerwünschte Neben-produkte, bei zu niedrigen Temperaturen sind die Umsätze unwirtschaftlich niedrig. Bevorzugt wird ein Temperaturbereich von 200 bis 400°C eingehalten.

Als Trägergas können beispielsweise Argon oder Stickstoff, bevorzugt Stickstoff eingesetzt werden. Der Druck beträgt bei der Umsetzung dabei vorzugsweise 0,05 bis 5 bar. Insbesondere wird bei Umgebungsdruck (Normaldruck) gearbeitet. Der Anteil des Inertgases am Reaktorfeed kann 0 bis 90 Mol-%, vorzugsweise 50 bis 85 Mol-% betragen.

Die Katalysatorbelastung, angegeben als Menge an N-(2-Hydroxyalkyl)carbonsäureamid pro Raumeinheit Katalysator und pro Zeiteinheit, kann 0,5 bis 30 Mol/l h, vorzugsweise 1 bis 15 Mol/l h betragen. Bei zu niedrigen Belastungen erhält man nur schlechte Raum-Zeit-Ausbeuten, bei zu hohen Belastungen werden nur ungenügende Umsätze erzielt, wodurch sich der Aufwand der nachfolgenden Aufarbeitung vergrößert.

Als N-(2-Hydroxyalkyl)carbonsäureamide werden vorzugsweise die C₂₋₁₂- Alkylverbindungen, besonders bevorzugt C₂₋₆-Alkylverbindungen, insbesondere (2-Hydroxyethyl)pyrrolidon oder der Diether davon eingesetzt.

Die Erfindung wird nachstehend anhand von Beispielen näher erläutert.

### Herstellung der Katalysatoren

Katalysator A: 500 g LDK10 (USY, Uetikon, Mod. 5,2) werden mit 35 g Walcocel® von Wolf/Walsrode und 470 ml Wasser 1 h im Kneter verdichtet und bei 80 bar Preßdruck zu 2 mm Strängen verformt. Die Stränge werden 16 h bei 110°C getrocknet und 16 h bei 500°C kalziniert.

Katalysator B: 160 g H-ZSM-5 (VAW, Mod. 28,6) werden mit 40 g Aerosil®-200 von Degussa 5 % Kartoffelstärke und 185 g Wasser 45 Minuten im Kneter verdichtet, danach bei einem Preßdruck von 90 bar zu 2 mm Strängen verformt, 16 h bei 110°C getrocknet und 16 h bei 500°C kalziniert.

Katalysator C: 120 g H-ZSM-5 (Uetikon, Mod. 57,4) werden mit 120 g Aerosil®-200, 5 % Kartoffelstärke und 200 g Wasser 45 Minuten im Kneter verdichtet, danach bei einem Preßdruck von 110 bar zu 2 mm Strängen verformt, 16 h bei 110°C getrocknet und 16 h bei 500°C kalziniert.

Katalysator D: 700 g NH₄-ZSM-5 (Conteka, Mod. 140) werden mit 7 % Walocel®, 330 g Wasser und 2 % NH₄OH Lösung 45 Minuten im Kneter verdichtet, danach bei einem Preßdruck von 90 bar zu 2 mm Strängen verformt, 16 h bei 110°C getrocknet und 16 h bei 500°C kalziniert.

Katalysator E: 800 g Na-Y Zeolith (BASF, Mod. 3,3) werden mit einer 20 % NH₄OH Lösung (Verhältnis 1:5) 2 h bei 80°C unter Rühren ausgetauscht. Das Produkt wird mit Wasser nitratfrei gewaschen, bei 110°C getrocknet und 2 h bei 590°C kalziniert. Diese Prozedur wird dreimal wiederholt. 610 g des so erhaltenen Produktes werden in 2N HNO₃ (Verhältnis 1:6) 3 h bei 95°C gerührt, mit heißem Wasser gewaschen, bei 110°C getrocknet und 4 h bei 530°C kalziniert. Das so erhaltene Produkt ist gemäß dem XRD-Spektrum ein Zeolith des Faujasit-Typs. Die Elementaranalyse ergab ein Modul von 149. 200 g dieses Produktes werden mit 8 % Walocel® von Wolf/Wolfsrode und 220 g Wasser 45 Minuten im Kneter verdichtet, bei einem Preßdruck von 100 bar zu 2 mm Strängen verformt, bei 110°C getrocknet und 16 h bei 500°C kalziniert.

Katalysator F: 240 g H-ZSM-5 (BASF, Mod. 386) werden mit Pural® von Condea, 2 % Ameisensäure und 180 g Wasser 45 Minuten im Kneter verdichtet, danach bei einem Preßdruck von 70 bar zu 2 mm Strängen verformt, 16 h bei 110°C getrocknet und 16 h bei 500°C kalziniert.

Katalysator G: 100 ml Stränge von Katalysator E werden mit einer Lösung von 30 g Kaliumacetat in 300 ml Wasser 24 h bei 80°C gerührt. Das Produkt wird abfiltriert, mit heißem Wasser gewaschen, bei 110°C getrocknet und 16 h bei 500°C kalziniert.

Katalysator H: 240 g Borsilikalit (BASF, Mod. 340) werden mit 105 g Aerosil®-200, 5 % Kartoffelstärke und 200 g Wasser 45 Minuten im Kneter verdichtet, danach bei einem Preßdruck von 70 bar zu 2 mm Strängen verformt, 16 h bei 110°C getrocknet und 16 h bei 500°C kalziniert. 30 g des so erhaltenen Produktes werden mit einer Lösung aus 3 g Kaliumcarbonat und 18 g Wasser imprägniert, bei 60°C und bei 110°C getrocknet und 2 h bei 540°C kalziniert.

Katalysator J: 240 g ZSM-5 (BASF, Mod. 386) werden mit 160 g Pural® (Verhältnis 60:40), 2 % Ameisensäure und 180 g Wasser 45 Minuten im Kneter verdichtet, bei einem Preßdruck von 70 bar zu 2 mm Strängen verformt, bei 110°C getrocknet und 16 h bei 500°C kalziniert. 100 ml des so erhaltenen Produktes werden mit einer Lösung aus 29,44 g Kaliumacetat und 300 ml Wasser 24 h bei 80°C behandelt, das erhaltene Produkt bei 110°C getrocknet und 16 h bei 500°C kalziniert.

### Dehydratisierung

### Allgemeine Versuchsbeschreibung

Die Versuche wurden in einem elektrisch beheizten, senkrechten Rohrreaktor mit einem inneren Durchmesser von 20 mm durchgeführt, in welchem die Katalysatorformlinge (30 ml) als Schüttung eingebracht wurden. Als Ver-dampfungszone für die von oben aufgegebene flüssige Ausgangsverbindung diente eine oberhalb der Katalysatorzone angeordnete Schüttung (50 ml) von Quarzringen. Die Temperatur in der Katalysatorzone wurde mittels eines zentrisch angeordneten, axial verschiebbaren Thermoelements gemessen und geregelt. Die Meßspitze des Thermoelements befand sich während des Betriebs der Apparatur in der Mitte der Katalysatorschüttung. Als Trägergasstrom wurde Reinst-Stickstoff getrennt von oben zudosiert. Der Produktstrom wurde unten am Reaktor abgenommen und durch einen Rohrschlangenkühler vollständig kondensiert und mittels Gaschromatographie mit internem Standard analysiert. Die angegebenen Resultate wurden aus mehrstündigen

Bilanzierungen nach Erreichen eines konstanten Betriebs ermittelt. Die Standardbedingungen waren: 350°C, Normaldruck, Belastung HEP 2,3 Mol/l h, Stickstoff 10 Mol/l h.

### Beispiele 1 bis 6 (nicht dotierte Katalysatoren)

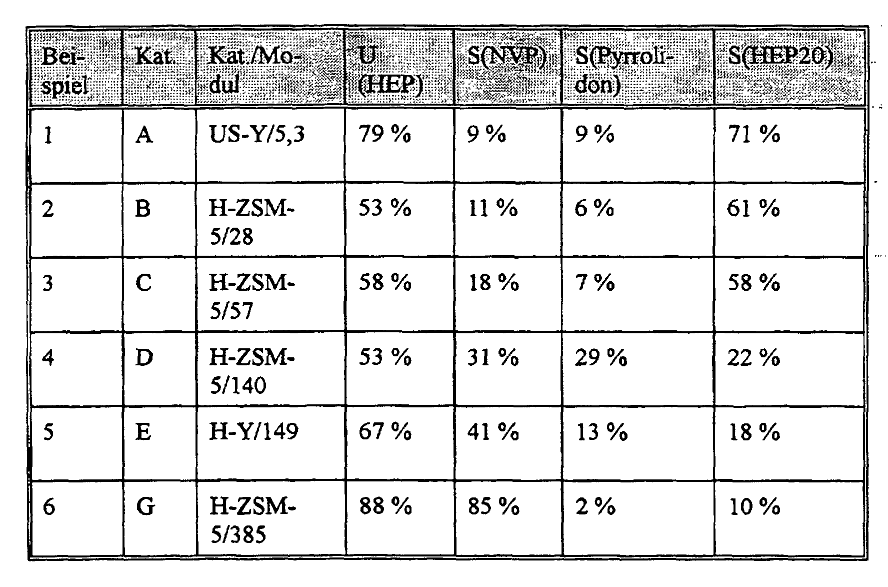

### Beispiele 7 und 8 (Kaliumdotierung)

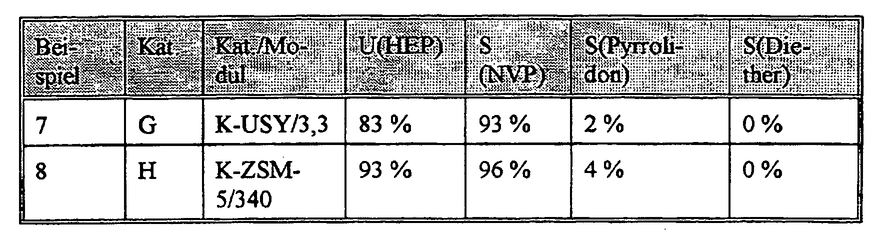

### Beispiele 9 bis 11 (Änderung der Katalysatorbelastung)

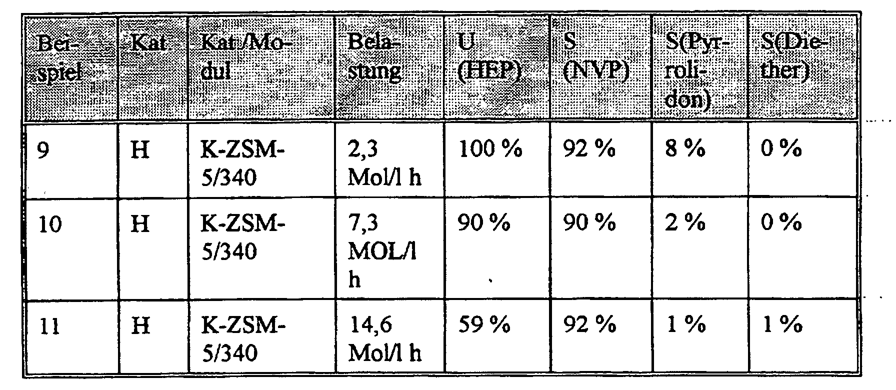

Wie aus den Ergebnissen der Beispiele 9 bis 11 hervorgeht, ist die Selektivität in bezug aufNVP in weiten Belastungsbereichen nahezu konstant.

### Beispiel 12 (Dehydratisierung des Diethers)

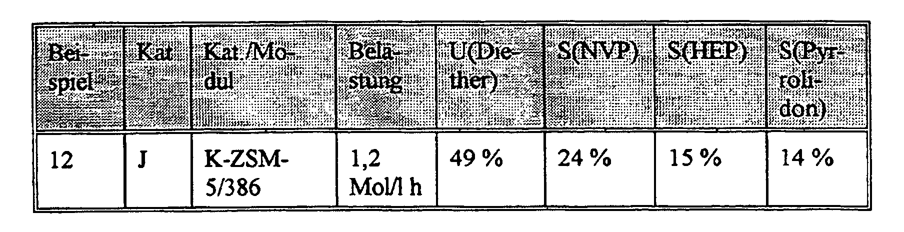

Die Eignung der beschriebenen Katalysatorsysteme zur Umwandlung des Diethers in NVP, entsprechend einer Rückführung des Diethers in einem kontinuierlichen Prozess, ist möglich, wie aus den Ergebnissen von Beispiel 12 hervorgeht. Es wurde unter Standardbedingungen gearbeitet, mit dem Unterschied, daß die Temperatur 400°C betrug.

## Patentansprüche

1. Verfahren zur Herstellung von N-Alkenylcarbonsäureamiden durch Dehydratisierung von N-(2-Hydroxyalkyl)carbonsäureamiden und/oder Diethern davon in Gegenwart eines Katalysators, **dadurch gekennzeichnet, daß** als Katalysator Molekularsiebe, insbesondere Zeolithe eingesetzt werden, wobei das Verfahren kontinuierlich durchgeführt wird und die nicht dehydratisierten N-(2-Hydroxyalkyl)carbonsäureamide und die als Nebenprodukte gebildeten Diether in die Dehydratisierung zurückgeführt werden.

2. Verfahren nach Anspruch 1, **dadurch gekennzeichnet, daß** in den Molekularsieben das Modul mindestens 140 beträgt.

3. Verfahren nach Anspruch 1 oder 2, **dadurch gekennzeichnet, daß** als Katalysatoren Zeolithe vom Typ ZSM5 (MFI), Y (FAU), LDK10 oder Silikalit eingesetzt werden.

4. Verfahren nach einem der Ansprüche 1 bis 3, **dadurch gekennzeichnet, daß** der Katalysator mit mindestens einer Alkalimetallverbindung oder Erdalkalimetallverbindung dotiert ist.

5. Verfahren nach einem der Ansprüche 1 bis 4, **dadurch gekennzeichnet, daß** der Katalysator als Formkörper in einem stationären oder bewegten Katalysatorbett eingesetzt wird.

6. Verfahren nach einem der Ansprüche 1 bis 5, **dadurch gekennzeichnet, daß** die Dehydratisierung in der Gasphase, gegebenenfalls in Gegenwart eines Trägergases, durchgeführt wird.

7. Verfahren nach einem der Ansprüche 1 bis 6, **dadurch gekennzeichnet, daß** die Dehydratisierung kontinuierlich durchgeführt wird.

8. Verfahren nach Anspruch 7, **dadurch gekennzeichnet, daß** nicht dehydratisierte N-(2-Hydroxyalkyl)carbonsäureamide und/oder als Nebenprodukte gebildete Diether davon in die Dehydratisierung zurückgeführt werden.

9. Verfahren nach einem der Ansprüche 1 bis 8, **dadurch gekennzeichnet, daß** (2-Hydroxyethyl)pyrrolidon oder Diether davon dehydratisiert werden.

## Claims

1. A process for preparing N-alkenylcarboxamides by dehydration of N-(2-hydroxyalkyl)carboxamides and/or diethers thereof in the presence of a catalyst, wherein the catalyst used is a molecular sieve, in particular a zeolite, and the process is carried out continuously and the N-(2-hydroxyalkyl)carboxamides which have not been dehydrated and the diethers formed as by-products are recirculated to the dehydration.

2. A process as claimed in claim 1, wherein the modulus of the molecular sieve is at least 140.

3. A process as claimed in claim 1 or 2, wherein the catalyst used is a zeolite of the type ZSM5 (MFI), Y (FAU), LDK10 or silicalite.

4. A process as claimed in any of claims 1 to 3, wherein the catalyst is doped with at least one alkali metal compound or alkaline earth metal compound.

5. A process as claimed in any of claims 1 to 4, wherein the catalyst is used as shaped bodies in a static or agitated catalyst bed.

6. A process as claimed in any of claims 1 to 5, wherein the dehydration is carried out in the gas phase, if desired in the presence of a carrier gas.

7. A process as claimed in any of claims 1 to 6, wherein the dehydration is carried out continuously.

8. A process as claimed in claim 7, wherein N-(2-hydroxyalkyl)carboxamides which have not been dehydrated and/or diethers thereof formed as by-products are recirculated to the dehydration.

9. A process as claimed in any of claims 1 to 8, wherein (2-hydroxyethyl)pyrrolidone or the diether thereof is dehydrated.

## Revendications

1. Procédé pour la préparation d'amides d'acides N-alcényl-carboxyliques par déshydratation d'amides d'acides N-(2-hydroxy-alkyl)carboxyliques et/ou de diéthers de ces derniers en présence d'un catalyseur, **caractérisé en ce qu'**on met en oeuvre, à titre de catalyseur, des tamis moléculaires, en particulier des zéolithes, le procédé étant mis en oeuvre en continu et les amides d'acides N-(2-hydroxyalkyl)carboxyliques non soumis à une déshydratation ainsi que les diéthers obtenus sous la forme de sous-produits étant renvoyés dans la déshydratation.

2. Procédé selon la revendication 1, **caractérisé en ce que**, dans les tamis moléculaires, le module s'élève à au moins 140.

3. Procédé selon la revendication 1 ou 2, **caractérisé en ce qu'**on met en oeuvre, à titre de catalyseurs, des zéolithes des types ZSM5 (MFI), Y (FAU), LDK10 ou de la silicalite.

4. Procédé selon l'une quelconque des revendications 1 à 3, **caractérisé en ce que** le catalyseur est dopé avec au moins un composé de métal alcalin ou un composé de métal alcalino-terreux.

5. Procédé selon l'une quelconque des revendications 1 à 4, **caractérisé en ce qu'**on met en oeuvre le catalyseur sous la forme de corps moulés dans un lit de catalyseur stationnaire ou en mouvement.

6. Procédé selon l'une quelconque des revendications 1 à 5, **caractérisé en ce qu'**on effectue la déshydratation en phase gazeuse, le cas échéant en présence d'un gaz porteur.

7. Procédé selon l'une quelconque des revendications 1 à 6, **caractérisé en ce qu'**on effectue la déshydratation en continu.

8. Procédé selon la revendication 7, **caractérisé en ce qu'**on renvoie dans la déshydratation les amides d'acides N-(2-hydroxyalkyl)-carboxyliques qui n'ont pas été déshydratés et/ou leurs diéthers obtenus sous la forme de sous-produits.

9. Procédé selon l'une quelconque des revendications 1 à 8, **caractérisé en ce qu'**on soumet à une déshydratation la (2-hydroxyéthyl)-pyrrolidone ou ses diéthers.
